# EUROPEAN PATENT APPLICATION

(11) **EP 4 174 180 A1**
(43) Date of publication of application: **03.05.2023**
(21) Application number: 21849156.1
(22) Date of filing: 27.07.2021
(51) Int. Cl.: C12N 15/31, C12Q 1/04, C12Q 1/686, C12Q 1/689, G01N 33/53

(54) **POLYNUCLEOTIDE, POLYNUCLEOTIDE SET, METHOD FOR DETECTING PORPHYROMONAS GINGIVALIS, METHOD FOR ASSESSING PERIODONTAL DISEASE SUSCEPTIBILITY, PORPHYROMONAS GINGIVALIS DETECTION KIT, AND PERIODONTAL DISEASE SUSCEPTIBILITY ASSESSMENT KIT**

(30) Priority: 28.07.2020 JP 2020127517
(71) Applicant: Mitsui Chemicals, Inc., Tokyo 104-0028 (JP)
(72) Inventor: MATOISHI, Kaori, Sodegaura-shi, Chiba 299-0265 (JP)
(74) Representative: Hoffmann Eitle
(86) International application number: PCT/JP2021/027765
(87) International publication number: WO 2022/025066

(57) **Abstract**

A polynucleotide of any one of the following: (1a) a polynucleotide including a base sequence of not less than 15 consecutive bases in base sequence A of any one of SEQ ID NO:1 to SEQ ID NO:9; (1b) a polynucleotide that hybridizes with a polynucleotide including the base sequence complementary to base sequence A, under stringent conditions; (1c) a polynucleotide including a base sequence having a sequence identity of not less than 80% to base sequence A; or (1d) a polynucleotide including a base sequence that is the same as base sequence A except that one or several bases are deleted, substituted, inserted, and/or added.

## Description

### Technical Field

The present disclosure relates to a polynucleotide, a polynucleotide set, a method of detecting *Porphyromonas gingivalis,* a method of evaluating susceptibility to periodontal disease, a kit for detecting *Porphyromonas gingivalis,* and a kit for evaluating susceptibility to periodontal disease.

### Background Art

Since *Porphyromonas gingivalis* is frequently detected in the buccal cavity of patients with periodontal disease, *Porphyromonas gingivalis* is known as a causative bacterium of periodontal disease. *Porphyromonas gingivalis* has the fimbrial gene *fimA,* and the fimbrial gene *fimA* can be divided into the following six types: type I, type Ib, type II, type III, type IV, and type V. Among the six types, *fimA* type II is known to pose the highest risk of development of periodontal disease.

Nucleic acid amplification reaction is widely applied to detection of causative microorganisms in the medical field and the food field since the reaction enables rapid, simple, and sensitive amplification of target nucleic acids. The presence of the *Porphyromonas gingivalis* can also be detected by performing nucleic acid amplification reaction for the base sequence of part of the fimbrial gene. Regarding the primers used for the nucleic acid amplification reaction, the following knowledge, for example, is available.

For example, Japanese Patent Application Laid-Open (JP-A) No. 2000-125874 discloses a detection method specific to *Porphyromonas gingivalis,* and also describes oligonucleotides used for the detection method.

Ji-Hoi Moon, et al., Development and evaluation of new primers for PCR-based identification of type II fimA of Porphyromonas gingivalis, FEMS Immunol Med Microbiol, 64:425-428, 2012. discloses a method capable of detecting *FimA* type II of *Porphyromonas gingivalis,* and also describes primers used for the detection.

Atsuo Amano, et al., Distribution of Porphyromonas gingivalis Strains with fimA Genotypes in Periodontitis Patients, J Clin Microbiol, 37:1426-1430, 1999. discloses a method capable of detecting *FimA* type II of *Porphyromonas gingivalis,* and also describes primers used for the detection.

### SUMMARY OF INVENTION

### Technical Problem

However, no polynucleotide capable of efficiently detecting *Porphyromonas gingivalis* has been obtained in any of JP-ANo. 2000-125874; Ji-Hoi Moon, et al., Development and evaluation of new primers for PCR-based identification of type II fimA of Porphyromonas gingivalis, FEMS Immunol Med Microbiol, 64:425-428, 2012.; and Atsuo Amano, et al., Distribution of Porphyromonas gingivalis Strains with fimA Genotypes in Periodontitis Patients, J Clin Microbiol, 37:1426-1430, 1999.

The present disclosure was carried out in view of the above fact, and an object of the disclosure is to provide a polynucleotide capable of achieving a favorable initial amplification efficiency and a favorable nucleic acid amplification efficiency of the fimbrial gene (*fimA*) of *Porphyromonas gingivalis.*

### Solution to Problem

Specific means for solving the problem includes the following modes.
<1> A polynucleotide of any one of the following (1a) to (1d):
   (1a) a polynucleotide including a base sequence of not less than 15 consecutive bases in a base sequence of any one of SEQ ID NO:1 to SEQ ID NO:9;
   (1b) a polynucleotide that hybridizes with a polynucleotide including a base sequence complementary to a base sequence of any one of SEQ ID NO:1 to SEQ ID NO:9, under stringent conditions;
   (1c) a polynucleotide including a base sequence having a sequence identity of not less than 80% to a base sequence of any one of SEQ ID NO:1 to SEQ ID NO:9; or
   (1d) a polynucleotide including a base sequence that is the same as a base sequence of any one of SEQ ID NO:1 to SEQ ID NO:9 except that one or several bases are deleted, substituted, inserted, and/or added;
<2> The polynucleotide according to <1>, having a number of bases of from 15 bases to 35 bases.
<3> The polynucleotide according to <1> or <2>, wherein the (1a) includes a base sequence of not less than 15 consecutive bases in a base sequence of SEQ ID NO:3, SEQ ID NO: 7, or SEQ ID NO:9.
<4> The polynucleotide according to <1> or <2>, including a base sequence of not less than 15 consecutive bases in a base sequence of any one of SEQ ID NO:1 to SEQ ID NO: 9.
<5> A polynucleotide set including: a polynucleotide (F) of any one of the following (2a) to (2d); and a polynucleotide (R) of any one of the following (3a) to (3d):
   (2a) a polynucleotide including a base sequence of not less than 15 consecutive bases in a base sequence of any one of SEQ ID NO: 1 to SEQ ID NO:3;
   (2b) a polynucleotide that hybridizes with a polynucleotide including a base sequence complementary to a base sequence of any one of SEQ ID NO: 1 to SEQ ID NO:3, under stringent conditions;
   (2c) a polynucleotide including a base sequence having a sequence identity of not less than 80% to a base sequence of any one of SEQ ID NO: 1 to SEQ ID NO:3;
   (2d) a polynucleotide including a base sequence that is the same as a base sequence of any one of SEQ ID NO: 1 to SEQ ID NO:3 except that one or several bases are deleted, substituted, inserted, and/or added;
   (3a) a polynucleotide including a base sequence of not less than 15 consecutive bases in a base sequence of any one of SEQ ID NO:4 to SEQ ID NO:9;
   (3b) a polynucleotide that hybridizes with a polynucleotide including a base sequence complementary to a base sequence of any one of SEQ ID NO:4 to SEQ ID NO:9, under stringent conditions;
   (3c) a polynucleotide including a base sequence having a sequence identity of not less than 80% to a base sequence of any one of SEQ ID NO:4 to SEQ ID NO:9;
   (3d) a polynucleotide including a base sequence that is the same as a base sequence of any one of SEQ ID NO:4 to SEQ ID NO:9 except that one or several bases are deleted, substituted, inserted, and/or added;
<6> The polynucleotide set according to <5>, wherein the polynucleotide (F) and the polynucleotide (R) each independently have a number of bases of from 15 bases to 35 bases.
<7> The polynucleotide set according to <5> or <6>,
   wherein
   the (2a) includes a base sequence of not less than 15 consecutive bases in a base sequence of SEQ ID NO:3; and
   the (3a) includes a base sequence of not less than 15 consecutive bases in a base sequence of SEQ ID NO:7 or SEQ ID NO:9.
<8> The polynucleotide set according to <5> or <6>,
   wherein
   the polynucleotide (F) includes a base sequence of not less than 15 consecutive bases in a base sequence of any one of SEQ ID NO: 1 to SEQ ID NO:3; and
   the polynucleotide (R) includes a base sequence of not less than 15 consecutive bases in a base sequence of any one of SEQ ID NON to SEQ ID NO:9.
<9> The polynucleotide set according to any one of <5> to <8>,
   wherein
   the polynucleotide (F) includes a base sequence of SEQ ID NO: 1, and the polynucleotide (R) includes a base sequence of SEQ ID NO:4, SEQ ID NO:5, SEQ ID NO:6, or SEQ ID NO:8;
   the polynucleotide (F) includes a base sequence of SEQ ID NO:2, and the polynucleotide (R) includes a base sequence of SEQ ID NO:4, SEQ ID NO:5, or SEQ ID NO:7; or
   the polynucleotide (F) includes a base sequence of SEQ ID NO:3, and the polynucleotide (R) includes a base sequence of SEQ ID NO:4.
<10> The polynucleotide set according to any one of <5> to <9>, for detecting *Porphyromonas gingivalis.*
<11> The polynucleotide set according to any one of <5> to <9>, for evaluating susceptibility to periodontal disease.
<12> A method of detecting *Porphyromonas gingivalis,* the method including a nucleic acid amplification reaction step using the polynucleotide set according to any one of <5> to <9>.
<13> A method of evaluating susceptibility to periodontal disease, the method including a nucleic acid amplification reaction step using the polynucleotide set according to any one of <5> to <9>.
<14> A kit for detecting *Porphyromonas gingivalis,* the kit including the polynucleotide set according to any one of <5> to <9>.
<15> A kit for evaluating susceptibility to periodontal disease, the kit including the polynucleotide set according to any one of <5> to <9>.

### Advantageous Effects of Invention

According to the disclosure, a polynucleotide capable of achieving a favorable initial amplification efficiency and a favorable nucleic acid amplification efficiency of the fimbrial gene (*fimA*) of *Porphyromonas gingivalis* can be provided.

### BRIEF DESCRIPTION OF DRAWING

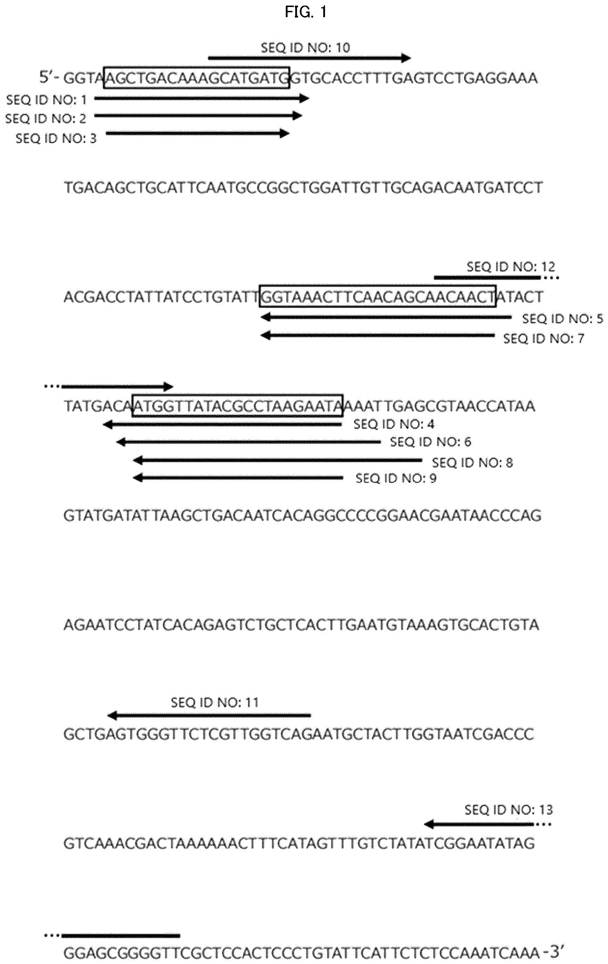

Fig. 1 shows a base sequence including the fimbrial gene *fimA* type II of *Porphyromonas gingivalis* and the vicinity thereof, and the positions of the base sequences of the polynucleotides used in Examples and Comparative Examples, in this base sequence. In the figure, each base sequence with a SEQ ID NO represented by a right arrow indicates that the base sequence is on the strand of the base sequence described in Fig. 1, and that this base sequence is used as a forward primer in the nucleic acid amplification reaction. Each base sequence with a SEQ ID NO represented by a left arrow indicates that the base sequence is on the complementary strand of the base sequence described in Fig. 1, and that this base sequence is used as a reverse primer in the nucleic acid amplification reaction. In the figure, "5'-" represents the 5'-end side, and "3'-" represents the 3'-end side. In the figure, the base sequences each enclosed in a rectangular frame represent, from the top, a base sequence common to SEQ ID NO: 1 to SEQ ID NO:3, a base sequence common to SEQ ID NO:5 and SEQ ID NO:7, and a base sequence common to SEQ ID NO:4, SEQ ID NO:6, SEQ ID NO:8, and SEQ ID NO:9. In the figure, SEQ ID NO: 10 and SEQ ID NO: 13 are depicted to illustrate possible positions of annealing of the base sequences of SEQ ID NO: 10 and SEQ ID NO: 13 in the nucleic acid amplification reaction, and the base sequences shown are different from the actual base sequences of SEQ ID NO: 10 and SEQ ID NO: 13.

### DESCRIPTION OF EMBODIMENTS

Embodiments according to the disclosure are described below in detail. However, the disclosure is not limited to the following embodiments. In the following disclosure, constituents (including element steps and the like) are not indispensable unless otherwise specified. Similarly, numerical values and their ranges do not limit the disclosure. In the disclosure, the term "step" may be either a step independent of other steps, or a step that is not clearly distinguishable from another step, as long as a desired purpose of the step can be achieved.

In the disclosure, a numerical range represented using "to" includes the numerical values described before and after the "to" as the minimum value and maximum value, respectively.

In a stepwise description of numerical ranges in the disclosure, the upper limit or lower limit described for a certain numerical range may be replaced by the upper limit or lower limit of another numerical range in the stepwise description. In a numerical range described in the text, the upper limit or lower limit of the numerical range may be replaced by a value described in Examples.

In the disclosure, in cases where a composition contains a plurality of kinds of substances corresponding to a certain component, the content ratio of the component in the composition means the total content ratio of the plurality of kinds of substances present in the composition, unless otherwise specified.

### «Polynucleotide»

### (Base Sequence of Polynucleotide)

The polynucleotide of the disclosure is one of the following (1a) to (1d):
(1a) a polynucleotide including a base sequence of not less than 15 consecutive bases in the base sequence of any one of SEQ ID NO: 1 to SEQ ID NO:9;
(1b) a polynucleotide that hybridizes with a polynucleotide including a base sequence complementary to the base sequence of any one of SEQ ID NO: 1 to SEQ ID NO:9, under stringent conditions;
(1c) a polynucleotide including a base sequence having a sequence identity of not less than 80% to the base sequence of any one of SEQ ID NO: 1 to SEQ ID NO:9; or
(1d) a polynucleotide including a base sequence that is the same as the base sequence of any one of SEQ ID NO: 1 to SEQ ID NO:9 except that one or several bases are deleted, substituted, inserted, and/or added.

SEQ ID NO: 1: 5'-aagctgacaaagcatgatggt-3'
SEQ ID NO:2: 5'-aagctgacaaagcatgatgg-3'
SEQ ID NO:3: 5'-agctgacaaagcatgatg-3'
SEQ ID NO:4: 5'-tattcttaggcgtataaccattgt-3'
SEQ ID NO:5: 5'-atagttgttgctgttgaagtttacc-3'
SEQ ID NO:6: 5'-attttattcttaggcgtataaccattg-3'
SEQ ID NO:7: 5'-agttgttgctgttgaagtttacc-3'
SEQ ID NO:8: 5'-ctcaattttattcttaggcgtataaccat-3'
SEQ ID NO:9: 5'-tattcttaggcgtataaccat-3'

In cases where the polynucleotide is one of the following (1a) to (1d), a favorable initial amplification efficiency and a favorable nucleic acid amplification efficiency of the fimbrial gene (*fimA*) of *Porphyromonas gingivalis* can be achieved.
(1a) A polynucleotide including a base sequence of not less than 15 consecutive bases in the base sequence of any one of SEQ ID NO: 1 to SEQ ID NO:9.
(1b) A polynucleotide that hybridizes with a polynucleotide including a base sequence complementary to the base sequence of any one of SEQ ID NO:1 to SEQ ID NO:9, under stringent conditions.
(1c) A polynucleotide including a base sequence having a sequence identity of not less than 80% to the base sequence of any one of SEQ ID NO: 1 to SEQ ID NO:9.
(1d) A polynucleotide including a base sequence that is the same as the base sequence of any one of SEQ ID NO: 1 to SEQ ID NO:9 except that one or several bases are deleted, substituted, inserted, and/or added.

Although the action of the polynucleotide of the disclosure is unclear, the action can be assumed as follows.

Since the polynucleotide of the disclosure has an appropriate base length, the polynucleotide is capable of stably binding to a template DNA, and secondary structures are unlikely to be formed in the polynucleotide (for example, formation of a hairpin structure is unlikely). Since the polynucleotide of the disclosure has an appropriate GC content, the polynucleotide has an appropriate Tm value (that is, an appropriate melting temperature), so that binding to and dissociation from a template DNA can be easily controlled in each step of nucleic acid amplification reaction (that is, the heat denaturation step, the annealing step, and the complementary strand synthesis step). Since the polynucleotide of the disclosure does not have uneven distribution of bases therein, secondary structures (such as a hairpin structure) are unlikely to be formed in the polynucleotide, and binding to and dissociation from a template DNA can be easily controlled in each step of nucleic acid amplification reaction. Since the polynucleotide of the disclosure, especially the base sequence in its 3'-end side, shows high sequence complementarity to the base sequence of the template DNA, complementary strand synthesis can be easily initiated in the complementary strand synthesis step. Since the polynucleotide of the disclosure has low polynucleotide-polynucleotide complementarity, polynucleotide dimers or polynucleotide spatial structures are unlikely to be formed.

The polynucleotide of the disclosure is designed such that the polynucleotide enables nucleic acid amplification of a base sequence contained in the fimbrial gene (referred to as *fimA* in the disclosure) of *Porphyromonas gingivalis.*

Therefore, the polynucleotide of the disclosure allows the heat denaturation step, the annealing step, and the complementary strand synthesis step to proceed smoothly during the nucleic acid amplification reaction. Thus, the polynucleotide of the disclosure is capable of achieving a favorable initial amplification efficiency and a favorable nucleic acid amplification efficiency of the fimbrial gene (*fimA*) of *Porphyromonas gingivalis.*

In cases where the initial amplification efficiency is favorable, the nucleic acid amplification reaction can be carried out even with a sample containing only a small amount of nucleic acid of *Porphyromonas gingivalis.* In other words, the presence of *Porphyromonas gingivalis* can be sensitively detected. In cases where the nucleic acid amplification efficiency is favorable, quantification of the *Porphyromonas gingivalis* contained in a sample can be accurately carried out.

The disclosure is not limited by the assumed mechanisms described above.

In the disclosure, the polynucleotide including a base sequence of not less than 15 consecutive bases in the base sequence of any one of SEQ ID NO:1 to SEQ ID NO:9 is not limited as long as the polynucleotide is a polynucleotide including a base sequence of not less than 15 consecutive bases in the base sequence of any one of SEQ ID NO:1 to SEQ ID NO:9. In the disclosure, the polynucleotide including a base sequence of not less than 15 consecutive bases in the base sequence of any one of SEQ ID NO: 1 to SEQ ID NO:9 is preferably a polynucleotide including a base sequence of from 15 to 29 consecutive bases, more preferably a polynucleotide including a base sequence of from 18 to 23 consecutive bases, from the viewpoint of easily allowing the polynucleotide to anneal to the template DNA. In the disclosure, in the polynucleotide including a base sequence of not less than 15 consecutive bases in the base sequence of any one of SEQ ID NO: 1 to SEQ ID NO:9, the portion of the base sequence of not less than 15 consecutive bases is preferably the portion of a base sequence of not less than 15 consecutive bases in the 3'-end side of the base sequence of any one of SEQ ID NO: 1 to SEQ ID NO:9, from the viewpoint of easily allowing the complementary strand synthesis to be initiated in the complementary strand synthesis step. The polynucleotide including a base sequence of not less than 15 consecutive bases is preferably a polynucleotide including a base sequence of from 15 to 29 consecutive bases, more preferably a polynucleotide including a base sequence of from 18 to 23 consecutive bases, from the viewpoint of easily allowing the polynucleotide to anneal to the template DNA.

In the disclosure, the polynucleotide that hybridizes with a polynucleotide including the base sequence complementary to the base sequence of any one of SEQ ID NO: 1 to SEQ ID NO:9, under stringent conditions is not limited as long as it is a polynucleotide that hybridizes with a polynucleotide including the base sequence complementary to the base sequence of any one of SEQ ID NO: 1 to SEQ ID NO:9, under stringent conditions. The hybridization under stringent conditions in the disclosure means hybridization under the conditions of the annealing step of the nucleic acid amplification reaction. More specifically, the hybridization under stringent conditions means, for example, that hybridization occurs under the following conditions: a temperature of from 40°C to 70°C; 5 seconds to 2 minutes. The hybridization in the disclosure is not limited as long as two polynucleotide molecules are capable of forming hydrogen bonds between their bases. The two nucleotide molecules may also include a portion where their bases do not form pairs.

In the disclosure, the polynucleotide including a base sequence having a sequence identity of not less than 80% to the base sequence of any one of SEQ ID NO: 1 to SEQ ID NO:9 is not limited as long as the polynucleotide is a polynucleotide including a base sequence having a sequence identity of not less than 80% to the base sequence of any one of SEQ ID NO: 1 to SEQ ID NO:9. Examples of the method of calculating the sequence identity in the disclosure include a method employing CLUSTAL W, which is provided by Center for Information Biology and DDBJ, National Institute of Genetics, with default parameters. From the viewpoint of easily allowing the polynucleotide to anneal to the template DNA, the sequence identity in the disclosure is preferably not less than 80%, more preferably not less than 85%, still more preferably not less than 90%, still more preferably not less than 95%, particularly preferably 100%.

In the disclosure, the polynucleotide including a base sequence that is the same as the base sequence of any one of SEQ ID NO: 1 to SEQ ID NO:9 except that one or several bases are deleted, substituted, inserted, and/or added is not limited as long as the polynucleotide is a polynucleotide including a base sequence that is the same as the base sequence of any one of SEQ ID NO: 1 to SEQ ID NO:9 except that one or several bases are deleted, substituted, inserted, and/or added. In the disclosure, the range of the one or several bases is preferably from 1 to 5 bases, more preferably from 1 to 3 bases, particularly preferably 1 base or 2 bases, from the viewpoint of easily allowing the polynucleotide to anneal to the template DNA. In the polynucleotide of the disclosure including a base sequence that is the same as the base sequence of any one of SEQ ID NO: 1 to SEQ ID NO:9 except that one or several bases are deleted, substituted, inserted, and/or added, the portion of the base sequence in which one or several bases are deleted, substituted, inserted, and/or added is preferably the portion of a base sequence in the 5'-end side of the base sequence of any one of SEQ ID NO: 1 to SEQ ID NO:9, from the viewpoint of easily allowing the complementary strand synthesis to be initiated in the complementary strand synthesis step.

The number of bases of the polynucleotide of the disclosure is preferably not too small from the viewpoint of easily allowing stable binding to the template DNA, and preferably not too long from the viewpoint of reducing formation of secondary structures (for example, reducing formation of hairpin structures) in the polynucleotide. More specifically, the polynucleotide of the disclosure has a number of bases of preferably from 15 bases to 35 bases, more preferably from 18 bases to 29 bases, or may have a number of bases of from 20 bases to 25 bases.

The polynucleotide of the disclosure preferably includes a base sequence of not less than 15 consecutive bases in the base sequence of SEQ ID NO:3, SEQ ID NO: 7, or SEQ ID NO:9 from the viewpoint of achieving a favorable initial amplification efficiency and a favorable nucleic acid amplification efficiency. The polynucleotide of the disclosure more preferably includes the base sequence of SEQ ID NO:3, SEQ ID NO: 7, or SEQ ID NO:9 from the viewpoint of achieving a favorable initial amplification efficiency and a favorable nucleic acid amplification efficiency.

The polynucleotide of the disclosure preferably includes a base sequence of not less than 15 consecutive bases in the base sequence of any one of SEQ ID NO:1 to SEQ ID NO:9 from the viewpoint of achieving a favorable initial amplification efficiency and a favorable nucleic acid amplification efficiency. The polynucleotide of the disclosure more preferably includes the base sequence of any one of SEQ ID NO:1 to SEQ ID NO:9, still more preferably is the base sequence of any one of SEQ ID NO: 1 to SEQ ID NO:9, from the viewpoint of achieving a favorable initial amplification efficiency and a favorable nucleic acid amplification efficiency.

The polynucleotide of the disclosure is preferably a single-stranded polynucleotide from the viewpoint of, for example, use as a primer for nucleic acid amplification.

The base at the 5'-end of the polynucleotide of the disclosure may have, in the upstream side of the base, an arbitrary base sequence having a base length of from 1 to 10, an arbitrary compound, or the like attached thereto. The sequence in the base sequence, and the molecular weight, the type, and the like of the compound are not limited. This is because the upstream side of the base at the 5'-end of the polynucleotide hardly affects the complementary strand synthesis in the complementary strand synthesis step. The compound may be, for example, a fluorescent dye commonly used for labeling of nucleic acid.

### (Method of Designing Polynucleotide)

A base sequence of the *fimA* gene of *Porphyromonas gingivalis* is described in Fujiwara, T., Morishima, S., Takahashi, I. and Hamada, S., Molecular cloning and sequencing of the fimbrilin gene of Porphyromonas gingivalis strains and characterization of recombinant proteins, Biochem. Biophys. Res. Commun. 197 (1), 241-247 (1993), and also available from a known database (such as DDBJ or NCBI).

For identifying a base sequence specific to the *fimA* type II gene of *Porphyromonas gingivalis,* base sequence information of the *fimA* gene in various bacteria may be obtained from a database (such as DDBJ or NCBI), and alignment analysis may be carried out. Examples of the method of the alignment analysis include a method employing CLUSTAL W, which is provided by Center for Information Biology and DDBJ, National Institute of Genetics, with default parameters.

In the disclosure, a primer may be designed taking into account the length, GC content, and Tm value of the polynucleotide; complementarity between polynucleotides; secondary structures in the polynucleotide; and/or the like. The primers may be designed also by using commercially available software for primer designing, such as Primer Express (manufactured by Thermo Fisher Scientific Inc.) or GENETYX (manufactured by Genetyx Corporation).

### (Method of Producing Polynucleotide)

The polynucleotide of the disclosure may be synthesized by a known polynucleotide synthesis method such as the phosphoramidite method or H-phosphonate method using an automated DNA synthesizer or the like. Alternatively, the polynucleotide of the disclosure may be synthesized by custom synthesis by Thermo Fisher Scientific Inc., Eurofins Genomics K. K., or the like.

### <<Polynucleotide Set>>

The polynucleotide set of the disclosure may include a polynucleotide (F) of any one of the following (2a) to (2d); and a polynucleotide (R) of any one of the following (3a) to (3d):
(2a) A polynucleotide including a base sequence of not less than 15 consecutive bases in the base sequence of any one of SEQ ID NO: 1 to SEQ ID NO:3.
(2b) A polynucleotide that hybridizes with a polynucleotide including a base sequence complementary to the base sequence of any one of SEQ ID NO:1 to SEQ ID NO:3, under stringent conditions.
(2c) A polynucleotide including a base sequence having a sequence identity of not less than 80% to the base sequence of any one of SEQ ID NO: 1 to SEQ ID NO:3.
(2d) A polynucleotide including a base sequence that is the same as the base sequence of any one of SEQ ID NO: 1 to SEQ ID NO:3 except that one or several bases are deleted, substituted, inserted, and/or added.
(3a) A polynucleotide including a base sequence of not less than 15 consecutive bases in the base sequence of any one of SEQ ID NO:4 to SEQ ID NO:9.
(3b) A polynucleotide that hybridizes with a polynucleotide including the base sequence complementary to the base sequence of any one of SEQ ID NO:4 to SEQ ID NO:9, under stringent conditions.
(3c) A polynucleotide including a base sequence having a sequence identity of not less than 80% to the base sequence of any one of SEQ ID NO:4 to SEQ ID NO:9.
(3d) A polynucleotide including a base sequence that is the same as the base sequence of any one of SEQ ID NO:4 to SEQ ID NO:9 except that one or several bases are deleted, substituted, inserted, and/or added.

### (Polynucleotide (F))

In the disclosure, (F) represents a forward primer. In the disclosure, the polynucleotide including a base sequence of not less than 15 consecutive bases in the base sequence of any one of SEQ ID NO:1 to SEQ ID NO:3 is not limited as long as the polynucleotide is a polynucleotide including a base sequence of not less than 15 consecutive bases in the base sequence of any one of SEQ ID NO:1 to SEQ ID NO:3. In the disclosure, the polynucleotide including a base sequence of not less than 15 consecutive bases in the base sequence of any one of SEQ ID NO:1 to SEQ ID NO:3 is preferably a polynucleotide including a base sequence of from 15 to 21 consecutive bases, more preferably a polynucleotide including a base sequence of from 18 to 21 consecutive bases, from the viewpoint of easily allowing the polynucleotide to anneal to the template DNA. In the disclosure, in the polynucleotide including a base sequence of not less than 15 consecutive bases in the base sequence of any one of SEQ ID NO:1 to SEQ ID NO:3, the portion of the base sequence of not less than 15 consecutive bases is preferably the portion of a base sequence of not less than 15 consecutive bases in the 3'-end side of the base sequence of any one of SEQ ID NO:1 to SEQ ID NO:3, from the viewpoint of easily allowing the complementary strand synthesis to be initiated in the complementary strand synthesis step. The polynucleotide including a base sequence of not less than 15 consecutive bases is preferably a polynucleotide including a base sequence of from 15 to 21 consecutive bases, more preferably a polynucleotide including a base sequence of from 18 to 21 consecutive bases, from the viewpoint of easily allowing the polynucleotide to anneal to the template DNA.

In the disclosure, the polynucleotide that hybridizes with a polynucleotide including the base sequence complementary to the base sequence of any one of SEQ ID NO:1 to SEQ ID NO:3, under stringent conditions is not limited as long as it is a polynucleotide that hybridizes with a polynucleotide including the base sequence complementary to the base sequence of any one of SEQ ID NO:1 to SEQ ID NO:3, under stringent conditions. The hybridization under stringent conditions in the disclosure means hybridization under the conditions of the annealing step of the nucleic acid amplification reaction. More specifically, the hybridization under stringent conditions means, for example, that hybridization occurs under the following conditions: a temperature of from 40°C to 70°C; 5 seconds to 2 minutes. The hybridization in the disclosure is not limited as long as two polynucleotide molecules are capable of forming hydrogen bonds between their bases. The two nucleotide molecules may also include a portion where their bases do not form pairs.

In the disclosure, the polynucleotide including a base sequence having a sequence identity of not less than 80% to the base sequence of any one of SEQ ID NO: 1 to SEQ ID NO:3 is not limited as long as the polynucleotide is a polynucleotide including a base sequence having a sequence identity of not less than 80% to the base sequence of any one of SEQ ID NO: 1 to SEQ ID NO:3. Examples of the method of calculating the sequence identity in the disclosure include a method employing CLUSTAL W, which is provided by Center for Information Biology and DDBJ, National Institute of Genetics, with default parameters. From the viewpoint of easily allowing the polynucleotide to anneal to the template DNA, the sequence identity in the disclosure is preferably not less than 80%, more preferably not less than 85%, still more preferably not less than 90%, still more preferably not less than 95%, particularly preferably 100%.

In the disclosure, the polynucleotide including a base sequence that is the same as the base sequence of any one of SEQ ID NO: 1 to SEQ ID NO:3 except that one or several bases are deleted, substituted, inserted, and/or added is not limited as long as the polynucleotide is a polynucleotide including a base sequence that is the same as the base sequence of any one of SEQ ID NO: 1 to SEQ ID NO:3 except that one or several bases are deleted, substituted, inserted, and/or added. In the disclosure, the range of the one or several bases is preferably from 1 to 5 bases, more preferably from 1 to 3 bases, particularly preferably 1 base or 2 bases, from the viewpoint of easily allowing the polynucleotide to anneal to the template DNA. In the polynucleotide of the disclosure including a base sequence that is the same as the base sequence of any one of SEQ ID NO: 1 to SEQ ID NO:3 except that one or several bases are deleted, substituted, inserted, and/or added, the portion of the base sequence in which one or several bases are deleted, substituted, inserted, and/or added is preferably the portion of a base sequence in the 5'-end side of the base sequence of any one of SEQ ID NO: 1 to SEQ ID NO:3, from the viewpoint of easily allowing the complementary strand synthesis to be initiated in the complementary strand synthesis step.

### (Polynucleotide (R))

In the disclosure, (R) represents a reverse primer. In the disclosure, the polynucleotide including a base sequence of not less than 15 consecutive bases in the base sequence of any one of SEQ ID NO:4 to SEQ ID NO:9 is not limited as long as the polynucleotide is a polynucleotide including a base sequence of not less than 15 consecutive bases in the base sequence of any one of SEQ ID NO:4 to SEQ ID NO:9. In the disclosure, the polynucleotide including a base sequence of not less than 15 consecutive bases in the base sequence of any one of SEQ ID NO:4 to SEQ ID NO:9 is preferably a polynucleotide including a base sequence of from 15 to 29 consecutive bases, more preferably a polynucleotide including a base sequence of from 18 to 21 consecutive bases, from the viewpoint of easily allowing the polynucleotide to anneal to the template DNA. In the disclosure, in the polynucleotide including a base sequence of not less than 15 consecutive bases in the base sequence of any one of SEQ ID NO:4 to SEQ ID NO:9, the portion of the base sequence of not less than 15 consecutive bases is preferably the portion of a base sequence of not less than 15 consecutive bases in the 3'-end side of the base sequence of any one of SEQ ID NO:4 to SEQ ID NO:9, from the viewpoint of easily allowing the complementary strand synthesis to be initiated in the complementary strand synthesis step. The polynucleotide including a base sequence of not less than 15 consecutive bases is preferably a polynucleotide including a base sequence of from 15 to 29 consecutive bases, more preferably a polynucleotide including a base sequence of from 18 to 21 consecutive bases, from the viewpoint of easily allowing the polynucleotide to anneal to the template DNA.

In the disclosure, the polynucleotide that hybridizes with a polynucleotide including the base sequence complementary to the base sequence of any one of SEQ ID NO:4 to SEQ ID NO:9, under stringent conditions is not limited as long as it is a polynucleotide that hybridizes with a polynucleotide including the base sequence complementary to the base sequence of any one of SEQ ID NO:4 to SEQ ID NO:9, under stringent conditions. The hybridization under stringent conditions in the disclosure means hybridization under the conditions of the annealing step of the nucleic acid amplification reaction. More specifically, the hybridization under stringent conditions means, for example, that hybridization occurs under the following conditions: a temperature of from 40°C to 70°C; 5 seconds to 2 minutes. The hybridization in the disclosure is not limited as long as two polynucleotide molecules are capable of forming hydrogen bonds between their bases. The two nucleotide molecules may also include a portion where their bases do not form pairs.

In the disclosure, the polynucleotide including a base sequence having a sequence identity of not less than 80% to the base sequence of any one of SEQ ID NO:4 to SEQ ID NO:9 is not limited as long as the polynucleotide is a polynucleotide including a base sequence having a sequence identity of not less than 80% to the base sequence of any one of SEQ ID NO:4 to SEQ ID NO:9. Examples of the method of calculating the sequence identity in the disclosure include a method employing CLUSTAL W, which is provided by Center for Information Biology and DDBJ, National Institute of Genetics, with default parameters. From the viewpoint of easily allowing the polynucleotide to anneal to the template DNA, the sequence identity in the disclosure is preferably not less than 80%, more preferably not less than 85%, still more preferably not less than 90%, still more preferably not less than 95%, particularly preferably 100%.

In the disclosure, the polynucleotide including a base sequence that is the same as the base sequence of any one of SEQ ID NO:4 to SEQ ID NO:9 except that one or several bases are deleted, substituted, inserted, and/or added is not limited as long as the polynucleotide is a polynucleotide including a base sequence that is the same as the base sequence of any one of SEQ ID NO:4 to SEQ ID NO:9 except that one or several bases are deleted, substituted, inserted, and/or added. In the disclosure, the range of the one or several bases is preferably from 1 to 5 bases, more preferably from 1 to 3 bases, particularly preferably 1 base or 2 bases, from the viewpoint of easily allowing the polynucleotide to anneal to the template DNA. In the polynucleotide of the disclosure including a base sequence that is the same as the base sequence of any one of SEQ ID NO:4 to SEQ ID NO:9 except that one or several bases are deleted, substituted, inserted, and/or added, the portion of the base sequence in which one or several bases are deleted, substituted, inserted, and/or added is preferably the portion of a base sequence in the 5'-end side of the base sequence of any one of SEQ ID NO:4 to SEQ ID NO:9, from the viewpoint of easily allowing the complementary strand synthesis to be initiated in the complementary strand synthesis step.

### (Combination of Polynucleotide (F) and Polynucleotide (R))

The combination of the polynucleotide (F) and the polynucleotide (R) in the polynucleotide set of the disclosure may be, for example, (2a) and (3a); (2a) and (3b); (2a) and (3c); (2a) and (3d); (2b) and (3a); (2b) and (3b); (2b) and (3c); (2b) and (3d); (2c) and (3a); (2c) and (3b); (2c) and (3c); (2c) and (3d); (2d) and (3a); (2d) and (3b); (2d) and (3c); or (2d) and (3d) described above.

In the polynucleotide set of the disclosure, the polynucleotide (F) and the polynucleotide (R) may each independently have a number of bases of from 15 bases to 35 bases.

The number of bases of each of the polynucleotide (F) and the polynucleotide (R) in the polynucleotide set of the disclosure is preferably not too small from the viewpoint of easily allowing stable binding to the template DNA, and preferably not too long from the viewpoint of reducing formation of secondary structures (for example, reducing formation of hairpin structures) in the polynucleotide. More specifically, the polynucleotide (F) and the polynucleotide (R) in the polynucleotide set of the disclosure each independently have a number of bases of preferably from 15 bases to 35 bases, more preferably from 18 bases to 29 bases, or may each independently have a number of bases of from 20 bases to 25 bases.

Preferably, in the polynucleotide set of the disclosure, the polynucleotide (F) includes a base sequence of not less than 15 consecutive bases in the base sequence of SEQ ID NO:3, and the polynucleotide (R) includes a base sequence of not less than 15 consecutive bases in the base sequence of SEQ ID NO:7 or SEQ ID NO:9, from the viewpoint of achieving a favorable initial amplification efficiency and a favorable nucleic acid amplification efficiency. More preferably, in the polynucleotide set of the disclosure, the polynucleotide (F) includes the base sequence of SEQ ID NO:3, and the polynucleotide (R) includes the base sequence of SEQ ID NO:7 or SEQ ID NO:9, from the viewpoint of achieving a favorable initial amplification efficiency and a favorable nucleic acid amplification efficiency.

Preferably, in the polynucleotide set of the disclosure, the polynucleotide (F) includes a base sequence of not less than 15 consecutive bases in the base sequence of any one of SEQ ID NO: 1 to SEQ ID NO:3, and the polynucleotide (R) includes a base sequence of not less than 15 consecutive bases in the base sequence of any one of SEQ ID NON to SEQ ID NO:9, from the viewpoint of achieving a favorable initial amplification efficiency and a favorable nucleic acid amplification efficiency. More preferably, in the polynucleotide set of the disclosure, the polynucleotide (F) includes the base sequence of any one of SEQ ID NO: 1 to SEQ ID NO:3, and the polynucleotide (R) includes the base sequence of any one of SEQ ID NON to SEQ ID NO:9, from the viewpoint of achieving a favorable initial amplification efficiency and a favorable nucleic acid amplification efficiency. Still more preferably, the base sequence of the polynucleotide (F) is any one of SEQ ID NO: 1 to SEQ ID NO:3, and the base sequence of the polynucleotide (R) is any one of SEQ ID NON to SEQ ID NO:9.

Preferably, in the polynucleotide set of the disclosure, the polynucleotide (F) includes the base sequence of SEQ ID NO: 1, and the polynucleotide (R) includes the base sequence of SEQ ID NO:4, SEQ ID NO:5, SEQ ID NO:6, or SEQ ID NO:8, from the viewpoint of achieving a favorable initial amplification efficiency and a favorable nucleic acid amplification efficiency. More preferably, in the polynucleotide set of the disclosure, the base sequence of the polynucleotide (F) is the base sequence of SEQ ID NO: 1, and the base sequence of the polynucleotide (R) is the base sequence of SEQ ID NO:4, SEQ ID NO:5, SEQ ID NO:6, or SEQ ID NO:8, from the viewpoint of achieving a favorable initial amplification efficiency and a favorable nucleic acid amplification efficiency.

In a preferred mode other than those described above, in the polynucleotide set of the disclosure, the polynucleotide (F) includes the base sequence of SEQ ID NO:2, and the polynucleotide (R) includes the base sequence of SEQ ID NO:4, SEQ ID NO:5, or SEQ ID NO:7, from the viewpoint of achieving a favorable initial amplification efficiency and a favorable nucleic acid amplification efficiency. More preferably, in the polynucleotide set of the disclosure, the base sequence of the polynucleotide (F) is the base sequence of SEQ ID NO:2, and the base sequence of the polynucleotide (R) is the base sequence of SEQ ID NO:4, SEQ ID NO:5, or SEQ ID NO:7, from the viewpoint of achieving a favorable initial amplification efficiency and a favorable nucleic acid amplification efficiency.

In a preferred mode other than those described above, in the polynucleotide set of the disclosure, the polynucleotide (F) includes the base sequence of SEQ ID NO:3, and the polynucleotide (R) includes the base sequence of SEQ ID NO:4, from the viewpoint of achieving a favorable initial amplification efficiency and a favorable nucleic acid amplification efficiency. More preferably, in the polynucleotide set of the disclosure, the base sequence of the polynucleotide (F) is the base sequence of SEQ ID NO:3, and the base sequence of the polynucleotide (R) is the base sequence of SEQ ID NO:4, from the viewpoint of achieving a favorable initial amplification efficiency and a favorable nucleic acid amplification efficiency.

The polynucleotide (F) of the disclosure may be included in a polynucleotide set including the polynucleotide (F) of the disclosure and a polynucleotide (R) that is not a polynucleotide of the disclosure. The polynucleotide (R) of the disclosure may be included in a polynucleotide set including the polynucleotide (R) of the disclosure and a polynucleotide (F) that is not a polynucleotide of the disclosure. Since the base sequence of each polynucleotide of the disclosure is designed to enable achievement of a favorable initial amplification efficiency and a favorable nucleic acid amplification efficiency, the favorable initial amplification efficiency and the favorable nucleic acid amplification efficiency can be sufficiently achieved even by a set of a polynucleotide of the disclosure and a polynucleotide that is not a polynucleotide of the disclosure.

### «Polynucleotide Set for Detecting Porphyromonas gingivalis»

The polynucleotide set of the disclosure can be used for detecting *Porphyromonas gingivalis.* The polynucleotide set for detecting *Porphyromonas gingivalis* of the disclosure is preferably for detecting *Porphyromonas gingivalis FimA* type II, from the viewpoint of enabling detection of *Porphyromonas gingivalis FimA* type II. The polynucleotide set for detecting *Porphyromonas gingivalis* of the disclosure is more preferably for specific detection of *Porphyromonas gingivalis FimA* type II, from the viewpoint of enabling specific detection of *Porphyromonas gingivalis FimA* type II.

As described above, *Porphyromonas gingivalis* can be divided into several types depending on the type of the fimbriae. Depending on the type of the fimbriae, the base sequence of the fimbrial gene varies. The base sequences of the polynucleotide (F) and the polynucleotide (R) in the polynucleotide set of the disclosure include a sequence specific to the fimbrial gene *fimA* type II of *Porphyromonas gingivalis.* Therefore, in nucleic acid amplification reaction using the polynucleotide (F) and the polynucleotide (R) of the polynucleotide set of the disclosure, annealing hardly occurs to *Porphyromonas gingivalis* fimbrial genes of the types other than *fimA* type II.

The base sequence of the fimbrial gene *fimA* of *Porphyromonas gingivalis FimA* type II has variation due to mutation of bases. The base sequences of the polynucleotide (F) and the polynucleotide (R) in the polynucleotide set of the disclosure are base sequences in especially conservative regions in the fimbrial gene *fimA* type II of *Porphyromonas gingivalis.* Therefore, in nucleic acid amplification reaction using the polynucleotide (F) and the polynucleotide (R) in the polynucleotide set of the disclosure, annealing to the *Porphyromonas gingivalis* fimbrial gene of *fimA* type II easily occurs, so that the nucleic acid amplification reaction can be efficiently carried out.

In the disclosure, detection of a target can be said to be successful in cases where at least one copy of nucleic acid is amplified by subjecting the later-described sample to a nucleic acid amplification reaction such as polymerase chain reaction (PCR) or its applied technique real-time PCR using the polynucleotide set of the disclosure as primers.

The polynucleotide set for detecting *Porphyromonas gingivalis* of the disclosure may further include the later-described TaqMan probe.

### «Polynucleotide Set for Evaluating Susceptibility to Periodontal Disease»

The polynucleotide set of the disclosure can be used for evaluating susceptibility to periodontal disease. Since *Porphyromonas gingivalis* is a causative bacterium of periodontal disease, the polynucleotide set of the disclosure, which is capable of detecting *Porphyromonas gingivalis,* can be used for evaluating susceptibility to periodontal disease.

In the disclosure, the evaluation of susceptibility to periodontal disease means either evaluation of the possibility that a patient is already suffering from periodontal disease, or evaluation of the possibility that a patient may suffer from periodontal disease in the future. In the disclosure, a patient can be said to be susceptible to periodontal disease in cases where at least one copy of nucleic acid is amplified by subjecting the later-described sample to a nucleic acid amplification reaction such as polymerase chain reaction (PCR) or its applied technique real-time PCR using the polynucleotide set of the disclosure as primers.

The polynucleotide set for evaluating susceptibility to periodontal disease of the disclosure is preferably a polynucleotide set for evaluating susceptibility to periodontal disease using *Porphyromonas gingivalis FimA* type II as an index since *Porphyromonas gingivalis FimA* type II is the type that poses the highest risk of development of periodontal disease.

The polynucleotide set for evaluating susceptibility to periodontal disease of the disclosure may also include the later-described TaqMan probe.

### <<Method of Detecting Porphyromonas gingivalis>>

The method of detecting *Porphyromonas gingivalis* of the disclosure may include a nucleic acid amplification reaction step using the polynucleotide set of the disclosure.

### (Nucleic Acid Amplification Reaction Step)

The nucleic acid amplification reaction step in the disclosure is not limited as long as the polynucleotide set of the disclosure is used, and may be carried out by a known method.

The reaction solution used in the nucleic acid amplification reaction step in the disclosure may be a solution prepared by mixing, for example, the later-described sample or nucleic acid extracted from the sample; the polynucleotide set of the disclosure; a nucleic acid-synthesizing enzyme (such as a DNA polymerase); deoxyribonucleoside triphosphates (dNTPs); water; a buffer; and optionally, a fluorescent reagent or fluorescently labeled probe. In the reaction solution, the amount of the sample or the nucleic acid extracted from the sample, the concentration of the polynucleotide set of the disclosure, the type and the concentration of the nucleic acid-synthesizing enzyme, the types and the concentrations of the deoxyribonucleoside triphosphates, the type and the concentration of the buffer, and the like may be appropriately set by those skilled in the art.

The nucleic acid amplification reaction step in the disclosure may further include a heat denaturation step, an annealing step, and a complementary strand synthesis step. The temperature, the number of cycles, and the time in each of the heat denaturation step, annealing step, and complementary strand synthesis step may be appropriately set by those skilled in the art. In the nucleic acid amplification reaction step in the disclosure, the annealing step and the complementary strand synthesis step may be carried out at the same time under the same conditions. In other words, the nucleic acid amplification reaction step may be 2-step PCR, in which each cycle is carried out by the heat denaturation step; and the annealing step and the complementary strand synthesis step.

In the nucleic acid amplification reaction step in the disclosure, real-time PCR is preferably carried out using a known real-time PCR apparatus from the viewpoint of achieving a rapid and highly quantitative reaction.

Real-time PCR is a method in which amplification of nucleic acid by PCR is monitored over time to quantify a target nucleic acid based on the amplification rate. This method may be carried out usually using an apparatus specially designed for real-time PCR, which apparatus includes a thermal cycler and a spectrofluorometer integrated therein.

In cases where real-time PCR is carried out in the nucleic acid amplification step, specific examples of details of the method of the real-time PCR include the following. First, PCR is carried out using, as a standard, serial dilutions of a nucleic acid whose amount is known, and the amount of amplification product is measured for each number of cycles. Subsequently, the number of cycles at which a certain amount of amplification product is obtained (Ct value; threshold cycle) within the range where the amplification of the nucleic acid exponentially occurs is plotted on the abscissa, and the initial amount of the nucleic acid is plotted on the ordinate, to prepare a calibration curve. A sample with an unknown concentration is also subjected to the reaction under the same conditions, to determine the Ct value. Using this value and the calibration curve, the amount of the target nucleic acid in the sample can be calculated.

Real-time PCR can be generally divided into methods using a fluorescent reagent and methods using a fluorescently labeled probe, depending on the detection method for the nucleic acid amplification product. Examples of the methods using a fluorescent reagent include the intercalator method, which uses a primer pair together with an intercalator (such as SYBR Green I) that is a compound which emits fluorescence upon binding to a double-stranded DNA. Examples of the methods using a fluorescently labeled probe include: the TaqMan method, which uses a TaqMan probe, molecular beacon probe, or cycling probe; the molecular beacon method; and the cycling probe method. In the nucleic acid amplification reaction step of the disclosure, the intercalator method is preferably used. Alternatively, from the viewpoint of enabling highly specific detection of the nucleic acid amplification product, the TaqMan method using a TaqMan probe is preferred.

The TaqMan method is a method that uses a polynucleotide probe (that is, a TaqMan probe) whose 5'-end is modified with a fluorescent substance such as FAM (Fluorescein) or HEX (Hexachlorofluorescein), and whose 3'-end is modified with a quencher substance such as TAMRA, NFQ (Non Fluorescent Quencher), or EQ (Eclipse Quencher). The TaqMan probe may be further modified with MGB (Minor Groove Binder) in order to realize a high melting temperature (Tm value) by forming a stable double-stranded structure with the template DNA. In the TaqMan method, the TaqMan probe specifically hybridizes with the target nucleic acid in the complementary strand synthesis step of PCR. As the synthesis of the complementary strand proceeds, the TaqMan probe is degraded, resulting in release of the fluorescent substance and hence an increase in the amount of fluorescence in the PCR solution. Since the increase in the amount of fluorescence can be used as an index of amplification of the target nucleic acid, the nucleic acid amplification can be simply detected in real time. Except for the use of the polynucleotide set described above, the real-time PCR method may be carried out based on a method known to those skilled in the art, using a commercially available real-time PCR kit or real-time PCR apparatus in accordance with an instruction provided by the manufacturer. Examples of the real-time PCR apparatus used in the nucleic acid amplification reaction step of the disclosure include Step One, manufactured by Thermo Fisher Scientific Inc., Thermal Cycler Dice Real Time System II, manufactured by Takara Bio Inc., and Rotor-Gene Q, manufactured by QIAGEN.

In the method of detecting *Porphyromonas gingivalis* of the disclosure, for the purpose of quantifying the *Porphyromonas gingivalis,* the calibration curve may be prepared using, as a standard, genomic DNA of the bacterial strain to be detected, or the calibration curve may be prepared using, as a standard, a plasmid or the like including the nucleic acid fragment to be amplified by the polynucleotide set of the disclosure. The plasmid used as a standard (standard plasmid) is not limited as long as the plasmid includes at least one nucleic acid fragment to be amplified by the polynucleotide set of the disclosure. More specifically, real-time PCR is carried out using the standard serial dilutions to prepare a calibration curve, and, based on the obtained calibration curve, the copy number of nucleic acid amplified from the sample is quantified. By this, the number of bacterial cells of *Porphyromonas gingivalis* in the sample can be quantified.

### (Sample and Template DNA)

The sample used in the method of detecting *Porphyromonas gingivalis* of the disclosure, which sample is used in the nucleic acid amplification reaction step, is not limited as long as the sample potentially includes *Porphyromonas gingivalis* as the detection target. Examples of the sample include saliva, dental plaque (supragingival dental plaque or subgingival dental plaque), tongue coating, and gingival exudate, collected from the oral cavity of a living body. The sample may also be blood such as plasma or serum, or urine. The saliva may be saliva discharged from the oral cavity, or a saliva mixture obtained by gargling with water and discharging. The dental plaque may be collected, for example, by brushing of the tooth surface using a dental scaler, cotton swab, brush, or the like, or by insertion of a paper point. The tongue coating may be collected using a brush, cotton swab, gauze, or the like. The gingival exudate may be collected by inserting a paper point or the like into the gingival crevice.

Regarding the template DNA used in the nucleic acid amplification reaction in the disclosure, the collected sample may be used as it is as the template DNA, or nucleic acid extracted from the sample may be used as the template DNA. The extraction of the nucleic acid from the sample may be carried out by the same method as a known nucleic acid extraction method. For example, a known method such as the SDS method, phenol method, or ethanol method may be applied to a sample such as saliva, or plaque on a paper point. Alternatively, a commercially available DNA extraction kit such as NucreoSpin (registered trademark) Blood (manufactured by QIAGEN); an automated DNA extractor; or the like may be used.

The method of detecting *Porphyromonas gingivalis* of the disclosure may be, for example, a method including:
providing a reaction solution containing: a sample obtained from a living body; a nucleic acid-synthesizing enzyme; and the polynucleotide set of the disclosure;
subjecting the reaction solution to nucleic acid amplification reaction; and
detecting nucleic acid amplified by the nucleic acid amplification reaction;
wherein detection of the amplified nucleic acid indicates the presence of *Porphyromonas gingivalis* in the sample.
Details of this reaction solution are as described for the reaction solution in the nucleic acid amplification reaction step.

The detection of *Porphyromonas gingivalis* may also be carried out based on the amount of the nucleic acid detected in the detection method. The detection of the amount of the nucleic acid can be carried out by measuring the fluorescence intensity of a fluorescent dye bound to the amplified nucleic acid, or by measuring a fluorescence intensity that can be an index of the nucleic acid amplification reaction as in the case of the TaqMan probe described later. Detection of a larger amount of nucleic acid suggests the presence of a larger amount of *Porphyromonas gingivalis* in the sample. The detection of the amplified nucleic acid may also be carried out during the nucleic acid amplification reaction. For example, by real-time PCR, the amplified nucleic acid can be detected in parallel with the nucleic acid amplification reaction. The detection may also be detection by detecting the fluorescence intensity.

### <<Method of Evaluating Susceptibility to Periodontal Disease>>

The method of evaluating susceptibility to periodontal disease of the disclosure may include a nucleic acid amplification reaction step using the polynucleotide set of the disclosure. The nucleic acid amplification reaction step in the method of evaluating susceptibility to periodontal disease of the disclosure may be in the same mode as the nucleic acid amplification reaction step in the <<Method of Detecting *Porphyromonas gingivalis*>>.

Based on the copy number of the amplified nucleic acid obtained by the nucleic acid amplification reaction step, susceptibility to periodontal disease can be evaluated. More specifically, a patient can be evaluated to be susceptible to periodontal disease in cases where at least one copy of nucleic acid is amplified by carrying out a nucleic acid amplification reaction such as polymerase chain reaction (PCR) or its applied technique real-time PCR using the polynucleotide set of the disclosure as primers. The larger the copy number of the amplified nucleic acid in the nucleic acid amplification reaction step, the higher the susceptibility to periodontal disease can be evaluated to be. The smaller the copy number of the amplified nucleic acid in the nucleic acid amplification reaction step, the lower the susceptibility to periodontal disease can be evaluated to be. In particular, in cases where nucleic acid of the *fimA* type II gene is amplified among the *fimA* genes, the susceptibility to periodontal disease can be evaluated to be high. Further, in cases where the nucleic acid of the *fimA* type II gene is amplified in a larger amount than the nucleic acid of other types of the *fimA* gene (for example, the *fimA* type I gene), the susceptibility to periodontal disease can be evaluated to be especially high.

The method of evaluating susceptibility to periodontal disease of the disclosure is preferably a method of evaluating susceptibility to periodontal disease using *Porphyromonas gingivalis FimA* type II as an index since *Porphyromonas gingivalis FimA* type II is the type that poses the highest risk of development of periodontal disease.

The method of evaluating susceptibility to periodontal disease of the disclosure may be, for example, a method including:
providing a reaction solution containing: a sample obtained from a living body; a nucleic acid-synthesizing enzyme; and the polynucleotide set of the disclosure;
subjecting the reaction solution to nucleic acid amplification reaction; and
detecting nucleic acid amplified by the nucleic acid amplification reaction;
wherein detection of the amplified nucleic acid indicates susceptibility to periodontal disease in the sample.
Details of this reaction solution are as described for the reaction solution in the nucleic acid amplification reaction step.

The evaluation of susceptibility to periodontal disease may also be carried out based on the amount of the nucleic acid detected in the evaluation method. The detection of the amount of the nucleic acid can be carried out by measuring the fluorescence intensity of a fluorescent dye bound to the amplified nucleic acid, or by measuring a fluorescence intensity that can be an index of the nucleic acid amplification reaction as in the case of the TaqMan probe described later. A larger amount of the detected nucleic acid suggests higher susceptibility to periodontal disease. The detection of the amplified nucleic acid may also be carried out during the nucleic acid amplification reaction. For example, by real-time PCR, the amplified nucleic acid can be detected in parallel with the nucleic acid amplification reaction. The detection may also be detection by detecting the fluorescence intensity.

### <<Kit for Detecting Porphyromonas gingivalis>>

The kit for detecting *Porphyromonas gingivalis* of the disclosure is not limited as long as the kit includes the polynucleotide set of the disclosure.

If necessary, the kit for detecting *Porphyromonas gingivalis* of the disclosure may also include: a reagent for nucleic acid extraction; a PCR reagent such as a PCR buffer, dNTPs, or DNA polymerase; a labeling substance; sterile water; a reference sample (such as a reference strain); a molecular weight marker; an instruction; or the like.

### <<Kit for Evaluating Susceptibility to Periodontal Disease>>

The kit for evaluating susceptibility to periodontal disease of the disclosure is not limited as long as the kit includes the polynucleotide set of the disclosure.

If necessary, the kit for evaluating susceptibility to periodontal disease of the disclosure may also include: a reagent for nucleic acid extraction; a PCR reagent such as a PCR buffer, dNTPs, or DNA polymerase; a labeling substance; sterile water; a reference sample (such as a reference strain); a molecular weight marker; an instruction; or the like.

### <<Other Modes>>

According to the disclosure, a method of diagnosing susceptibility to periodontal disease, including a polynucleotide set is provided.

According to the disclosure, a polynucleotide set for detecting *Porphyromonas gingivalis* is also provided.

According to the disclosure, a polynucleotide set for evaluating susceptibility to periodontal disease is also provided.

According to the disclosure, use of a polynucleotide set for detecting *Porphyromonas gingivalis* is also provided.

According to the disclosure, use of a polynucleotide set for evaluating susceptibility to periodontal disease is also provided.

According to the disclosure, use of a polynucleotide set in the production of a kit for detecting *Porphyromonas gingivalis* is also provided.

According to the disclosure, use of a polynucleotide set in the production of a kit for evaluating susceptibility to periodontal disease is also provided.

Regarding details of the polynucleotide set, the above description on the polynucleotide set according to the disclosure applies as it is also to these modes.

### EXAMPLES

The disclosure is described below more specifically by way of Examples. However, the disclosure is not limited to the following Examples as long as the spirit of the disclosure is not spoiled. Unless otherwise specified, "parts" is on a mass basis. Similarly, "%" is on a mass basis.

### «Example 1»

### (Synthesis of Polynucleotides)

Polynucleotides of the following base sequences were synthesized by outsourcing, and subjected to desalting purification. The corresponding position of each of the following polynucleotides in the *Porphyromonas gingivalis* gene is depicted in Fig. 1.
SEQ ID NO:1: 5'-aagctgacaaagcatgatggt-3' (forward primer)
SEQ ID NO:2: 5'-aagctgacaaagcatgatgg-3' (forward primer)
SEQ ID NO:3: 5'-agctgacaaagcatgatg-3' (forward primer)
SEQ ID NO:4: 5'-tattcttaggcgtataaccattgt-3' (reverse primer)
SEQ ID NO:5: 5'-atagttgttgctgttgaagtttacc-3' (reverse primer)
SEQ ID NO:6: 5'-attttattcttaggcgtataaccattg-3' (reverse primer)
SEQ ID NO:7: 5'-agttgttgctgttgaagtttacc-3' (reverse primer)
SEQ ID NO:8: 5'-ctcaattttattcttaggcgtataaccat-3' (reverse primer)
SEQ ID NO:9: 5'-tattcttaggcgtataaccat-3' (reverse primer)

The sequence identities between the base sequences are, for example, as follows.
SEQ ID NO:4: SEQ ID NO:6 Sequence identity, 95%
SEQ ID NO:4: SEQ ID NO:8 Sequence identity, 87%
SEQ ID NO:6: SEQ ID NO:8 Sequence identity, 92%

### (Template DNA Extraction from Samples)

As a sample for a calibration curve, a purified culture liquid of the following reference strain of *Porphyromonas gingivalis FimA* type II was used. As clinical samples, gingival exudates collected from patients with periodontal disease were used.

### - Sample for Calibration Curve -

As a reference strain of *Porphyromonas gingivalis FimA* type II, *Porphyromonas gingivalis* JCM19600 (distributed from Microbe Division, RIKEN BioResource Research Center; Complete genome sequence of the bacterium *Porphyromonas gingivalis* TDC60, which causes periodontal disease., Watanabe T et al., 2011 J Bacteriol. 193(16):4259-4260) was used. The reference strain was cultured, and genomic DNA was extracted from 1 ml of purified culture liquid using a commercially available DNA extraction kit (NucreoSpin (registered trademark) Blood, manufactured by QIAGEN). The extracted genomic DNA was diluted to 1 ng/µl using measurement of the absorbance, and then further serially diluted to 0.1 ng/µl, 0.01 ng/µl, 0.001 ng/µl, and 0.0001 ng/µl, to provide template DNA. The number of bacterial cells of the reference strain of *Porphyromonas gingivalis FimA* type II per 1 ng of the genomic DNA was set to 3.9 × 10⁵.

### - Clinical Samples -

Human gingival exudate was collected as follows. Sample collection was carried out for the deepest periodontal pocket in the oral cavity. After removing the saliva in the vicinity of the periodontal pocket, a commercially available paper point (Morita Paper Point Short 04, manufactured by J. MORITA CORP.) was inserted into the periodontal pocket, and left to stand for 10 seconds, followed by removing the paper point. The collection operation was carried out for three patients with periodontal disease, to collect three kinds of clinical samples. From the three kinds of clinical samples, DNA was extracted to provide clinical sample 1, clinical sample 2, and clinical sample 3 as template DNAs using a commercially available DNA extraction kit (NucreoSpin (registered trademark) Blood, manufactured by QIAGEN).

### (Nucleic Acid Amplification Reaction for Template DNA by Real-Time PCR)

Using each template DNA obtained by the above method, real-time PCR was carried out using Step One, manufactured by Thermo Fisher Scientific Inc. The composition of the real-time PCR reaction solution was as follows.

### - Composition of Real-Time PCR Reaction Solution (Total Volume, 10 µl) -

Template DNA 1 µl
Forward primer of SEQ ID NO:1 (10 µM) 0.75 µl
Reverse primer of SEQ ID NO:5 (10 µM) 0.75 µl
PowerUp^{™} SYBR (registered trademark) Green Master Mix5 µl
   (manufactured by Thermo Fisher Scientific Inc.)
Ultrapure water 2.5 µl

In the nucleic acid amplification reaction, pretreatment was carried out at 50°C for 2 minutes and 95°C for 2 minutes, and then 2-step PCR was carried out for 40 cycles, wherein each cycle was carried out by heat denaturation at 95°C for 15 seconds, and annealing and complementary strand synthesis at 60°C for 1 minute. After the completion of the real-time PCR, nucleic acid was quantified by the standard curve method, and the threshold cycle was set by the crossing point method.

### - Method of Evaluating Calibration Curve -

After the nucleic acid amplification reaction using the sample for a calibration curve as the template DNA, a calibration curve was prepared. The calibration curve was automatically prepared for the DNA copy numbers of 2 × 10⁶, 2 × 10⁵, 2 × 10⁴, 2 × 10³, 2 × 10², and 2 × 10¹ by the above-described real-time PCR software. In the preparation of the calibration curve, the number of cycles at which a certain amount of amplification product was obtained (Ct value; threshold cycle) within the range where the amplification of the nucleic acid exponentially occurred was plotted on the abscissa, and the initial amount of the nucleic acid (log value) was plotted on the ordinate. For the calibration curve, the correlation coefficient R² (linearity), the value of the y-intercept, and the slope were determined. The results on the prepared calibration curve are shown in Table 1 together with the results in other Examples and Comparative Examples described later. Each SEQ ID NO in Table 1 represents the type of the polynucleotide used in each case.

The initial amplification efficiency was rated A to C based on the following criteria. The y-intercept of the calibration curve indicates the number of cycles of the real-time PCR at which the quantified value is 1. It can be said that the higher the value, the lower the initial amplification efficiency, so that a larger amount of nucleic acid is required for the quantification. In the disclosure, the initial amplification efficiency can be said to be excellent in cases where the initial amplification efficiency is rated A or B.
A: The y-intercept value of the calibration curve is from 33.9 to less than 39.0.
B: The y-intercept value of the calibration curve is from 39.0 to less than 42.0.
C: The y-intercept value of the calibration curve is not less than 42.0.

The nucleic acid amplification efficiency (%) was calculated according to the following calculation equation: nucleic acid amplification efficiency (%) = {10^{(-1/slope of the calibration curve).} -1} × 100. The nucleic acid amplification efficiency was rated A to C based on the following criteria. In cases where the nucleic acid amplification efficiency is low, a large amount of nucleic acid is required in the quantification, and moreover, non-specific amplification is likely to occur. In the disclosure, the nucleic acid amplification efficiency can be said to be excellent in cases where the nucleic acid amplification efficiency is rated A or B.
A: The value of the nucleic acid amplification efficiency (%) is from 90% to less than 110%.
B: The value of the nucleic acid amplification efficiency (%) is from 80% to less than 90%.
C: The value of the nucleic acid amplification efficiency (%) is less than 80%.

### - Method of Evaluating Specificity -

The products obtained by the nucleic acid amplification reaction using each of clinical sample 1 to clinical sample 3 as the template DNA were used for evaluation of the specificity. Using the Ct value of each of clinical sample 1 to clinical sample 3, and the calibration curve, the amount of nucleic acid in each clinical sample was calculated. The results on the specificity in clinical sample 1 to clinical sample 3 are shown in Table 2.

In the table, P.g represents *Porphyromonas gingivalis.* Each SEQ ID NO in the table represents the type of the polynucleotide used in each case. Each value in the table represents the copy number in each sample. For example, 2.6E+03 indicates that the result suggests the presence of 2.6×10³ copies of *Porphyromonas gingivalis* in the sample (that is, the number of bacterial cells of *Porphyromonas gingivalis* in the sample is 2.6 × 10³).

The specificity was rated A to C based on the following criteria. In the disclosure, specific detection of *Porphyromonas gingivalis FimA* type II can be said to be successful in cases where the specificity is rated A or B.
A: The copy number in clinical sample 1 is from 1 × 10³ to less than 1 × 10⁴;
   the copy number in clinical sample 2 is less than 2 × 10⁴; and
   the copy number in clinical sample 3 is 0.
B: The copy number in clinical sample 1 is from 1 × 10³ to less than 1 × 10⁴;
   the copy number in clinical sample 2 is less than 2×10⁴; and
   the copy number in clinical sample 3 is from more than 0 to less than 1×10².
C: Neither A nor B is satisfied.

### «Example 2»

Nucleic acid amplification reaction by real-time PCR, evaluation of the calibration curve, and evaluation of the specificity were carried out in the same manner as in Example 1 except that the primer of SEQ ID NO:2 was used as the forward primer, and that the primer of SEQ ID NO:4 was used as the reverse primer.

### «Example 3»

Nucleic acid amplification reaction by real-time PCR, evaluation of the calibration curve, and evaluation of the specificity were carried out in the same manner as in Example 1 except that the primer of SEQ ID NO:2 was used as the forward primer, and that the primer of SEQ ID NO:7 was used as the reverse primer.

### «Example 4»

Nucleic acid amplification reaction by real-time PCR, evaluation of the calibration curve, and evaluation of the specificity were carried out in the same manner as in Example 1 except that the primer of SEQ ID NO:1 was used as the forward primer, and that the primer of SEQ ID NO:6 was used as the reverse primer.

### «Example 5»

Nucleic acid amplification reaction by real-time PCR, evaluation of the calibration curve, and evaluation of the specificity were carried out in the same manner as in Example 1 except that the primer of SEQ ID NO:1 was used as the forward primer, and that the primer of SEQ ID NO:8 was used as the reverse primer.

### «Example 6»

Nucleic acid amplification reaction by real-time PCR, evaluation of the calibration curve, and evaluation of the specificity were carried out in the same manner as in Example 1 except that the primer of SEQ ID NO:2 was used as the forward primer, and that the primer of SEQ ID NO:5 was used as the reverse primer.

### «Example 7»

Nucleic acid amplification reaction by real-time PCR, evaluation of the calibration curve, and evaluation of the specificity were carried out in the same manner as in Example 1 except that the primer of SEQ ID NO:3 was used as the forward primer, and that the primer of SEQ ID NON was used as the reverse primer.

### <<Comparative Example 1>>

Nucleic acid amplification reaction by real-time PCR, evaluation of the calibration curve, and evaluation of the specificity were carried out in the same manner as in Example 1 except that the primer of SEQ ID NO:10 was used as the forward primer, and that the primer of SEQ ID NO:11 was used as the reverse primer. The base sequences of SEQ ID NO:10 and SEQ ID NO:11 are the base sequences of the primers for amplification of the *fimA* type II gene of *Porphyromonas gingivalis,* described in Ji-Hoi Moon, et al., Development and evaluation of new primers for PCR-based identification of type II fimA of Porphyromonas gingivalis, FEMS Immunol Med Microbiol, 64:425-428, 2012. The polynucleotides of SEQ ID NO:10 to SEQ ID NO:15 described below were similarly synthesized by outsourcing, and subjected to desalting purification.
SEQ ID NO:10: 5'-gcatgatggtactcctttga-3' (forward primer)
SEQ ID NO:11: 5'-ctgaccaacgagaacccact-3' (reverse primer)

### <<Comparative Example 2»

Nucleic acid amplification reaction by real-time PCR, evaluation of the calibration curve, and evaluation of the specificity were carried out in the same manner as in Example 1 except that the primer of SEQ ID NO:12 was used as the forward primer, and that the primer of SEQ ID NO:13 was used as the reverse primer. The base sequences of SEQ ID NO:12 and SEQ ID NO:13 are the base sequences of the primers for amplification of the *fimA* type II gene of *Porphyromonas gingivalis,* described in Atsuo Amano, et al., Distribution of Porphyromonas gingivalis Strains with fimA Genotypes in Periodontitis Patients, J Clin Microbiol, 37:1426-1430, 1999.
SEQ ID NO:12: 5'-acaactatacttatgacaatgg-3' (forward primer)
SEQ ID NO:13: 5'-aaccccgctccctgtattccga-3' (reverse primer)

### <<Reference Example 1»

Detection of the total number of bacterial cells of *Porphyromonas gingivalis* was carried out without distinguishing among the types of *Porphyromonas gingivalis FimA.* More specifically, nucleic acid amplification reaction by real-time PCR, preparation of the calibration curve, and evaluation of the specificity were carried out in the same manner as in Example 1 except that the primer of SEQ ID NO:14 was used as the forward primer, and that the primer of SEQ ID NO:15 was used as the reverse primer. The base sequences of SEQ ID NO:14 and SEQ ID NO:15 are base sequences designed based on the base sequences of the primers for detection of *Porphyromonas gingivalis,* described in Hiroshi Maeda, et al., Quantitative real-time PCR using TaqMan and SYBR Green for Actinobacillus actinomycetemcomitans, Porphyromonas gingivalis, Prevotella intermedia, tetQ gene and total bacteria, FEMS Immunol Med Microbiol, 39:81-86, 2003.
SEQ ID NO:14: 5'-tagcttgctaaggttgatgg-3' (forward primer)
SEQ ID NO:15: 5'-caagtgtatgcggttttagt-3' (reverse primer)

### «Evaluation Results»

### (Evaluation of Calibration Curves)

Table 1 shows the results obtained using genomic DNA of the reference strain of *Porphyromonas gingivalis FimA* type II and each polynucleotide, according to the - Method of Evaluating Calibration Curve -.

**[Table 1]**

| | Polynucleotide | R² of calibration curve | Y-intercept of calibration curve | Evaluation of initial amplification efficiency | Slope of calibration curve | Nucleic acid amplification efficiency (%) | Evaluation of nucleic acid amplification efficiency |
|---|---|---|---|---|---|---|---|
| Example 1 | SEQ ID NO: 1 | 1.000 | 37.2 | A | -3.52 | 92.2 | A |
| | SEQ ID NO:5 | | | | | | |
| Example 2 | SEQ ID NO:2 | 0.998 | 37.4 | A | -3.47 | 94.0 | A |
| | SEQ ID NO:4 | | | | | | |
| Example 3 | SEQ ID NO:2 | 0.996 | 34.0 | A | -3.61 | 90.0 | A |
| | SEQ ID NO:7 | | | | | | |
| Example 4 | SEQ ID NO: 1 | 0.998 | 38.1 | A | -3.90 | 80.6 | B |
| | SEQ ID NO:6 | | | | | | |
| Example 5 | SEQ ID NO: 1 | 0.998 | 39.7 | B | -3.90 | 80.4 | B |
| | SEQ ID NO:8 | | | | | | |
| Example 6 | SEQ ID NO:2 | 0.998 | 37.8 | A | -3.57 | 90.6 | A |
| | SEQ ID NO:5 | | | | | | |
| Example 7 | SEQ ID NO:3 | 1.000 | 37.1 | A | -3.58 | 90.3 | A |
| | SEQ ID NO:4 | | | | | | |
| Comparative Example 1 | SEQ ID NO:10 | 0.999 | 44.8 | C | -3.39 | 97.3 | A |
| | SEQ ID NO:11 | | | | | | |
| Comparative Example 2 | SEQ ID NO:12 | 1.000 | 42.3 | C | -4.30 | 70.8 | C |
| | SEQ ID NO:13 | | | | | | |

As shown in Table 1, Example 1 to Example 7 showed excellent results in both the evaluation of the initial amplification efficiency and the evaluation of the nucleic acid amplification efficiency. However, Comparative Example 1 and Comparative Example 2 showed poor results in both the evaluation of the initial amplification efficiency and the evaluation of the nucleic acid amplification efficiency.

### (Evaluation of Specificity)

Table 2 shows the results on the specificity, obtained according to the - Method of Evaluating Specificity - using the clinical samples and the polynucleotides. In Table 2, no data means that this test was not carried out.

**[Table 2]**

| | Type of P.g contained in sample | Reference Example 1 | Comparative Example 1 | Comparative Example 2 | Example 1 | Example 2 | Example 3 | Example 4 | Example 5 | Example 6 | Example 7 |
|---|---|---|---|---|---|---|---|---|---|---|---|
| | | SEQ ID NO:14 | SEQ ID NO:10 | SEQ ID NO:12 | SEQ ID NO:1 | SEQ ID NO:2 | SEQ ID NO:2 | SEQ ID NO:1 | SEQ ID NO:1 | SEQ ID NO:2 | SEQ ID NO:3 |
| | | SEQ ID NO:15 | SEQ ID NO:11 | SEQ ID NO:13 | SEQ ID NO:5 | SEQ ID NO:4 | SEQ ID NO:7 | SEQ ID NO:6 | SEQ ID NO:8 | SEQ ID NO:5 | SEQ ID NO:4 |
| Clinical sample 1 | *FimA* type II | 2.6E+03 | 7.6E+04 | 2.7E+03 | 5.5E+03 | 6.0E+03 | 4.0E+03 | 4.9E+03 | 4.3E+03 | 4.1E+03 | 3.8E+03 |
| Clinical sample 2 | Mixture of *FimA* type I and small amount of *FimA* type II | 1.1E+04 | 3.8E+05 | 7.7E+03 | 2.7E+02 | 3.3E+02 | 2.4E+02 | 2.7E+02 | 3.0E+02 | 1.3E+02 | 1.8E+02 |
| Clinical sample 3 | *FimA* type I | 1.2E+04 | no data | 1.4E+04 | 0.0E+00 | 0.0E+00 | 0.0E+00 | 0.0E+00 | 3.2E+01 | 0.0E+00 | 5.1E+00 |
| Evaluation of specificity | | | C | C | A | A | A | A | B | A | B |

Clinical sample 1 includes only *Porphyromonas gingivalis FimA* type II. Clinical sample 2 includes *Porphyromonas gingivalis FimA* type I and a small amount of *Porphyromonas gingivalis FimA* type II. Clinical sample 3 includes only *Porphyromonas gingivalis FimA* type I. As shown in Table 2, regarding the results on the specificity in the clinical samples (clinical sample 1 to clinical sample 3), the specificity was rated A or B in each of Example 1 to Example 7, indicating that *Porphyromonas gingivalis FimA* type II could be specifically detected. However, in Comparative Example 1 and Comparative Example 2, *Porphyromonas gingivalis FimA* type II could not be specifically detected.

### «Example 8»

### (Synthesis of TaqMan Probes)

Synthesis and HPLC purification of TaqMan probes of the following base sequences were carried out by outsourcing. More specifically, the TaqMan probe of SEQ ID NO: 16 was obtained by outsourcing to Thermo Fisher Scientific Inc., and the TaqMan probes of SEQ ID NO: 17 and SEQ ID NO18 were obtained by outsourcing to Eurofins Genomics K. K. In the sequences, both FAM (Fluorescein) and HEX (Hexachlorofluorescein) are fluorochrome labels for biolabeling; NFQ is an abbreviation for Non Fluorescent Quencher; MGB is an abbreviation for Minor Groove Binder; and EQ is an abbreviation for Eclipse Quencher.
SEQ ID NO: 16: 5'-FAM-acttcaacagcaacaactat-NFQ-MGB-3' (TaqMan probe)
SEQ ID NO: 17: 5'-HEX-atagttgttgctgttgaagt-MGB-EQ-3' (TaqMan probe)
SEQ ID NO: 18 :5'-HEX-cctacgacctattatcctgtat-MGB-EQ-3' (TaqMan probe)

### (Nucleic Acid Amplification Reaction for Template DNA by Real-Time PCR)

Using each template DNA described above, real-time PCR was carried out using Step One, manufactured by Thermo Fisher Scientific Inc. The composition of the real-time PCR reaction solution was as follows.

### - Composition of Real-Time PCR Reaction Solution (Total Volume, 10 µl) - Template DNA 1 µl

Forward primer of SEQ ID NO: 1 (10 µM) 0.75 µl
Reverse primer of SEQ ID NO:4 (10 µM) 0.75 µl
TaqMan probe of SEQ ID NO: 16 (1 µM) 2.5 µl
Probe qPCR Mix (manufactured by Takara Bio Inc.)5 µl

In the nucleic acid amplification reaction, pretreatment was carried out at 95°C for 30 seconds, and then 2-step PCR was carried out for 40 cycles, wherein each cycle was carried out by heat denaturation at 95°C for 5 seconds, and annealing and complementary strand synthesis at 60°C for 60 seconds. After the completion of the real-time PCR, nucleic acid was quantified by the standard curve method, and the threshold cycle was set by the crossing point method.

The method of evaluating the calibration curve in Example 8 was carried out in the same manner as the method of evaluating the calibration curve described in Example 1. More specifically, for the calibration curve, the correlation coefficient R² (linearity), the value of the y-intercept, and the slope were determined. Further, evaluation of the initial amplification efficiency, calculation of the nucleic acid amplification efficiency, and evaluation of the nucleic acid amplification efficiency were carried out. The results on the calibration curve are shown in Table 3 together with the results in other Examples described later. The SEQ ID NO of each polynucleotide in Table 3 represents the type of the polynucleotide used in each case.

The method of evaluating the specificity in Example 8 was carried out in the same manner as the method of evaluating the specificity described in Example 1. The results on the specificity in clinical sample 1 to clinical sample 3 are shown in Table 4. In the table, P.g represents *Porphyromonas gingivalis.* Each SEQ ID NO in the Table represents the type of the polynucleotide used in each case. Each value in the table represents the copy number in each sample. For example, 2.6E+03 indicates that the result suggests the presence of 2.6×10³ copies of *Porphyromonas gingivalis* in the sample (that is, the number of bacterial cells of *Porphyromonas gingivalis* in the sample is 2.6 × 10³).

### «Example 9»

Nucleic acid amplification reaction by real-time PCR, evaluation of the calibration curve, and evaluation of the specificity were carried out in the same manner as in Example 8 except that the probe of SEQ ID NO:17 was used as the TaqMan probe.

### «Example 10»

Nucleic acid amplification reaction by real-time PCR, evaluation of the calibration curve, and evaluation of the specificity were carried out in the same manner as in Example 8 except that the primer of SEQ ID NO: 1 was used as the forward primer, that the primer of SEQ ID NO:5 was used as the reverse primer, and that the probe of SEQ ID NO: 18 was used as the TaqMan probe.

### «Evaluation Results»

### (Evaluation of Calibration Curves)

Table 3 shows the results obtained using genomic DNA of the reference strain of *Porphyromonas gingivalis FimA* type II and each polynucleotide, according to the - Method of Evaluating Calibration Curve -.

**[Table 3]**

| | Polynucleotide | TaqMan probe | R² of calibration curve | Y-intercept of calibration curve | Evaluation of initial amplification efficiency | Slope of calibration curve | Nucleic acid amplification efficiency (%) | Evaluation of nucleic acid amplification efficiency |
|---|---|---|---|---|---|---|---|---|
| Example 8 | SEQ ID NO:1 | SEQ ID NO:16 | 0.999 | 38.5 | A | -3.12 | 109.3 | A |
| | SEQ ID NO:4 | | | | | | | |
| Example 9 | SEQ ID NO:1 | SEQ ID NO:17 | 0.996 | 40.8 | B | -3.41 | 96.5 | A |
| | SEQ ID NO:4 | | | | | | | |
| Example 10 | SEQ ID NO:1 | SEQ ID NO:18 | 0.998 | 41.0 | B | -3.26 | 102.8 | A |
| | SEQ ID NO:5 | | | | | | | |

As shown in Table 3, Example 8 to Example 10 showed excellent results in both the evaluation of the initial amplification efficiency and the evaluation of the nucleic acid amplification efficiency. In Example 8 to Example 10, the TaqMan probes of SEQ ID NO: 16 to SEQ ID NO: 18, respectively, could be confirmed to be probes applicable to the TaqMan method.

### (Evaluation of Specificity)

Table 4 shows the results on the specificity, obtained according to the - Method of Evaluating Specificity - using the clinical samples and the polynucleotides. The Reference Example 1 in Table 4 is the same as the Reference Example 1 described above.

**[Table 4]**

| | Type of P.g contained in sample | Reference Example 1 | Example 8 | Example 9 | Example 10 |
|---|---|---|---|---|---|
| | | SEQ ID NO:14 | SEQ ID NO:1 | SEQ ID NO:1 | SEQ ID NO:1 |
| | | SEQ ID NO:15 | SEQ ID NO:4 | SEQ ID NO:4 | SEQ ID NO:5 |
| | | - | SEQ ID NO:16 | SEQ ID NO:17 | SEQ ID NO:18 |
| Clinical sample 1 | *FimA* type II | 2.6E+03 | 2.1E+03 | 3.8E+03 | 4.0E+03 |
| Clinical sample 2 | Mixture of *FimA* type I and small amount of *FimA* type II | 1.1E+04 | 5.0E+01 | 2.7E+02 | 1.0E+02 |
| Clinical sample 3 | *FimA* type I | 1.2E+04 | 0.0E+00 | 0.0E+00 | 0.0E+00 |
| Evaluation of specificity | | | A | A | A |

As shown in Table 4, regarding the results on the specificity in the clinical samples (clinical sample 1 to clinical sample 3), the specificity was rated A in each of Example 8 to Example 10, indicating that *Porphyromonas gingivalis FimA* type II could be specifically detected.

Thus, in the Examples of the disclosure, polynucleotides capable of achieving a favorable initial amplification efficiency and a favorable nucleic acid amplification efficiency of the fimbrial gene (*fimA*) of *Porphyromonas gingivalis* could be provided. Further, in the Examples of the disclosure, polynucleotides capable of specifically detecting *Porphyromonas gingivalis FimA* type II could be provided. In contrast, Comparative Examples failed to provide such polynucleotides capable of achieving a favorable initial amplification efficiency and a favorable nucleic acid amplification efficiency of the fimbrial gene (*fimA*) of *Porphyromonas gingivalis,* and moreover, failed to provide polynucleotides capable of specifically detecting *Porphyromonas gingivalis FimA* type II.

The disclosure of Japanese patent application No. 2020-127517, filed on July 28, 2020, is incorporated herein by reference in its entirety.

All documents, patent applications, and technical standards described in the present description are incorporated herein by reference to the same extent as in cases where the individual documents, patent applications, and technical standards are specifically and individually described to be incorporated by reference.

### INDUSTRIAL APPLICABILITY

By carrying out nucleic acid amplification reaction using the polynucleotide of the disclosure as a primer, *Porphyromonas gingivalis* can be detected, or susceptibility to periodontal disease can be evaluated.

## Claims

1. A polynucleotide of any one of the following (1a) to (1d):
(1a) a polynucleotide comprising a base sequence of not less than 15 consecutive bases in a base sequence of any one of SEQ ID NO:1 to SEQ ID NO:9;
(1b) a polynucleotide that hybridizes with a polynucleotide comprising a base sequence complementary to a base sequence of any one of SEQ ID NO: 1 to SEQ ID NO:9, under stringent conditions;
(1c) a polynucleotide comprising a base sequence having a sequence identity of not less than 80% to a base sequence of any one of SEQ ID NO: 1 to SEQ ID NO:9; or
(1d) a polynucleotide comprising a base sequence that is the same as a base sequence of any one of SEQ ID NO: 1 to SEQ ID NO:9 except that one or several bases are deleted, substituted, inserted, and/or added.

2. The polynucleotide according to claim 1, having a number of bases of from 15 bases to 35 bases.

3. The polynucleotide according to claim 1 or claim 2, wherein the (1a) comprises a base sequence of not less than 15 consecutive bases in a base sequence of SEQ ID NO:3, SEQ ID NO: 7, or SEQ ID NO:9.

4. The polynucleotide according to claim 1 or claim 2, comprising a base sequence of not less than 15 consecutive bases in a base sequence of any one of SEQ ID NO: 1 to SEQ ID NO: 9.

5. A polynucleotide set comprising: a polynucleotide (F) of any one of the following (2a) to (2d); and a polynucleotide (R) of any one of the following (3a) to (3d):
(2a) a polynucleotide comprising a base sequence of not less than 15 consecutive bases in a base sequence of any one of SEQ ID NO:1 to SEQ ID NO:3;
(2b) a polynucleotide that hybridizes with a polynucleotide comprising a base sequence complementary to a base sequence of any one of SEQ ID NO: 1 to SEQ ID NO:3, under stringent conditions;
(2c) a polynucleotide comprising a base sequence having a sequence identity of not less than 80% to a base sequence of any one of SEQ ID NO: 1 to SEQ ID NO:3;
(2d) a polynucleotide comprising a base sequence that is the same as a base sequence of any one of SEQ ID NO: 1 to SEQ ID NO:3 except that one or several bases are deleted, substituted, inserted, and/or added;
(3a) a polynucleotide comprising a base sequence of not less than 15 consecutive bases in a base sequence of any one of SEQ ID NON to SEQ ID NO:9;
(3b) a polynucleotide that hybridizes with a polynucleotide comprising a base sequence complementary to a base sequence of any one of SEQ ID NON to SEQ ID NO:9, under stringent conditions;
(3c) a polynucleotide comprising a base sequence having a sequence identity of not less than 80% to a base sequence of any one of SEQ ID NON to SEQ ID NO:9;
(3d) a polynucleotide comprising a base sequence that is the same as a base sequence of any one of SEQ ID NON to SEQ ID NO:9 except that one or several bases are deleted, substituted, inserted, and/or added.

6. The polynucleotide set according to claim 5, wherein the polynucleotide (F) and the polynucleotide (R) each independently have a number of bases of from 15 bases to 35 bases.

7. The polynucleotide set according to claim 5 or claim 6, wherein
the (2a) comprises a base sequence of not less than 15 consecutive bases in a base sequence of SEQ ID NO: 3; and
the (3a) comprises a base sequence of not less than 15 consecutive bases in a base sequence of SEQ ID NO:7 or SEQ ID NO:9.

8. The polynucleotide set according to claim 5 or claim 6, wherein
the polynucleotide (F) comprises a base sequence of not less than 15 consecutive bases in a base sequence of any one of SEQ ID NO: 1 to SEQ ID NO:3; and
the polynucleotide (R) comprises a base sequence of not less than 15 consecutive bases in a base sequence of any one of SEQ ID NON to SEQ ID NO:9.

9. The polynucleotide set according to any one of claims 5 to 8, wherein
the polynucleotide (F) comprises a base sequence of SEQ ID NO: 1, and the polynucleotide (R) comprises a base sequence of SEQ ID NO:4, SEQ ID NO:5, SEQ ID NON, or SEQ ID NO: 8;
the polynucleotide (F) comprises a base sequence of SEQ ID NO:2, and the polynucleotide (R) comprises a base sequence of SEQ ID NO:4, SEQ ID NO:5, or SEQ ID NO:7; or
the polynucleotide (F) comprises a base sequence of SEQ ID NO:3, and the polynucleotide (R) comprises a base sequence of SEQ ID NO:4.

10. The polynucleotide set according to any one of claims 5 to 9, for detecting *Porphyromonas gingivalis.*

11. The polynucleotide set according to any one of claims 5 to 9, for evaluating susceptibility to periodontal disease.

12. A method of detecting *Porphyromonas gingivalis,* the method comprising a nucleic acid amplification reaction step using the polynucleotide set according to any one of claims 5 to 9.

13. A method of evaluating susceptibility to periodontal disease, the method comprising a nucleic acid amplification reaction step using the polynucleotide set according to any one of claims 5 to 9.

14. A kit for detecting *Porphyromonas gingivalis,* the kit comprising the polynucleotide set according to any one of claims 5 to 9.

15. A kit for evaluating susceptibility to periodontal disease, the kit comprising the polynucleotide set according to any one of claims 5 to 9.
